# EUROPEAN PATENT SPECIFICATION

(11) **EP 2 240 182 B1**
(45) Date of publication and mention of the grant of the patent: **01.06.2016**
(21) Application number: 09710362.6
(22) Date of filing: 12.02.2009
(51) Int. Cl.: A61K 31/593, A61K 31/59, A61P 3/08, A61P 3/10

(54) **TREATING HYPERGLYCEMIA WITH 25-HYDROXYVITAMIN D3 AND VITAMIN D**
BEHANDLUNG VON HYPERGLYKÄMIE MIT 25-HYDROXYVITAMIN D3 UND VITAMIN D
TRAITEMENT DE L'HYPERGLYCÉMIE AVEC DE LA 25-HYDROXY-VITAMINE D3 ET DE LA VITAMINE D

(30) Priority: 13.02.2008 US 28510 P; 26.02.2008 US 31671 P; 14.03.2008 US 36927 P; 15.03.2008 US 36928 P
(43) Date of publication of application: 20.10.2010
(73) Proprietor: DSM IP Assets B.V., 6411 TE Heerlen (NL)
(72) Inventor: BUCK, Neil, Robert, F-68220 Leymen (FR); LEUENBERGER, Bruno, H., CH-4123 Rheinfelden (CH); STOECKLIN, Elisabeth, CH-4144 Arlesheim (CH); URBAN, Kai, 79713 Bad Säckingen (DE); WOLFRAM, Swen, 79761 Waldshut-Tiengen (DE); CLAERHOUT, Wouter, CH-4102 Binningen (CH)
(74) Representative: Rabanus, Birgit
(86) International application number: PCT/EP2009/051640
(87) International publication number: WO 2009/101136

(56) References cited:
- US-A1- 2003 170 324
- ZITTERMANN ET AL: "Vitamin D and disease prevention with special reference to cardiovascular disease" PROGRESS IN BIOPHYSICS AND MOLECULAR BIOLOGY, PERGAMON PRESS, OXFORD, GB, vol. 92, no. 1, 1 September 2006 (2006-09-01), pages 39-48, XP025078429 ISSN: 0079-6107 [retrieved on 2006-09-01]
- GRANT ET AL: "Epidemiology of disease risks in relation to vitamin D insufficiency" PROGRESS IN BIOPHYSICS AND MOLECULAR BIOLOGY, PERGAMON PRESS, OXFORD, GB, vol. 92, no. 1, 1 September 2006 (2006-09-01), pages 65-79, XP025078432 ISSN: 0079-6107 [retrieved on 2006-09-01]
- TAI ET AL: "Vitamin D, glucose, insulin, and insulin sensitivity" NUTRITION, ELSEVIER INC, US, vol. 24, no. 3, 9 January 2008 (2008-01-09), pages 279-285, XP022453376 ISSN: 0899-9007
- DATABASE EMBASE [Online] ELSEVIER SCIENCE PUBLISHERS, AMSTERDAM, NL; October 1998 (1998-10), AYESHA I ET AL: "Effect of oral administration of vitamin D on glucose tolerance and insulin secretion in Type 2 diabetes mellitus" XP002525919 Database accession no. EMB-1999146146 & DIABETES, NUTRITION AND METABOLISM - CLINICAL AND EXPERIMENTAL 199810 IT, vol. 11, no. 5, October 1998 (1998-10), pages 261-265, ISSN: 0394-3402
- LICATA A A: "EFFECT OF CALCIFEDIOL AND CALCITRIOL ON GLUCOSE TOLERANCE AND INSULIN SECRETION IN TYPE II DIABETES MELLITUS" JOURNAL OF THE AMERICAN COLLEGE OF NUTRITION, AMERICAN COLLEGE OF NUTRION, WILMINGTON, NC, US, vol. 6, no. 5, 1 October 1987 (1987-10-01), page 436, XP009115789 ISSN: 0731-5724
- MOREY-HOLTON EMILY ET AL: "The hindlimb unloading rat model: literature overview, technique update and comparison with space flight data.", ADVANCES IN SPACE BIOLOGY AND MEDICINE 2005, vol. 10, 2005, pages 7-40, XP009168591, ISSN: 1569-2574

## Description

### FIELD OF THE INVENTION

The present invention relates to treating hyperglycemia in a human with 25-hydroxyvitamin D3 (calcifediol). Blood glucose is reduced to a level which is closer to normal than baseline. Vitamin D3 is used together with 25-hydroxyvitamin D3.

### Background of the Invention

Vitamin D (e.g., ergocalciferol and cholecalciferol) is a group of fat-soluble compounds defined by their biological activity. A deficiency of vitamin D causes rickets in children and osteomalacia in adults. But toxicity can occur after chronic intake of more than 100 times the recommended daily allowance (i.e., 5-15 µg or 200-600 IU vitamin D) for several months. For vitamin D, "The threshold for toxicity is 500 to 600 mcg/kg body weight per day. In general, adults should not consume more than three times the RDA for extended period of time" (Garrison & Somer, The Nutrition Desk Reference, Third Ed., McGraw-Hill, pg. 82, 1997). Hypercalcemia may occur at a blood concentration of 25-hydroxyvitamin D greater than 375 nmol/L. More recently, a safe upper level of Vitamin D was identified to be at least 250 µg/ day (10'000 IU) (Hathcock et al. Am.J Clin. Nutr. 85:6-18, 2007). Ingestion of such as a dietary supplement has been shown to result in a blood concentration of about 200 nmol/L 25-hydroxyvitamin D.

Vitamin D is a prohormone which has to be hydroxylated in the liver to produce 25-hydroxyvitamin D (calcifediol; 25-OH vitamin D; 25-OH D), which then undergoes another hydroxylation in the kidney and other tissues to produce 1,25-dihydroxyvitamin D, the active hormone form of vitamin D. 1,25-dihydroxyvitamin D is released into the blood, binds to vitamin D binding protein (DBP), and is transported to target tissues. Binding between 1,25-dihydroxyvitamin D and vitamin D receptor allows the complex to act as a transcription factor in the cell's nucleus.

Vitamin D deficiency may promote resorption of bone. It may also modulate function of the cardiovascular, immune, and muscular systems. Epidemiological studies find associations between vitamin D intake and its effect on blood pressure or glucose metabolism. The activity of vitamin D is under negative feedback control by parathyroid hormone.

Both Vitamin D and 25-OH D3 have been administered as pharmaceuticals in the past. Vitamin D, is of course widely available; 25-OH D3 was previously sold in the USA by Organon USA under the name "CALDEROL", but is currently on the FDA's list of discontinued drugs. It was a gelatine capsule containing corn oil and 25-OH D3.

A liquid form of 25-OH D3 is currently sold in Spain by FAES Farma under the name "HIDROFEROL" in an oil solution.

The combination of vitamin D and 25-OH D3 has been used in animal feed. 25-OH D3 for use in feed is commercially available from DSM under the name "ROVIMIX HY-D".

Tritsch et al. (US 2003/0170324) disclose a feed premix composition of at least 25-OH D3 in an amount between 5% and 50% (wt/wt) dissolved in oil and an antioxidant, an agent encapsulating droplets of 25-OH D3 and oil, and a nutritional additive (e.g., Vitamin D3). The premix may be added to poultry, swine, canine, or feline food. This composition stabilizes 25-OH D3 against oxidation.

Simoes-Nunes et al. (US 2005/0064018) discloses adding a combination of 25-OH Vitamin D3 and Vitamin D3 to animal feed. In particular, about 10 µg/kg to about 100µg/kg of 25-OH Vitamin D3 and about 200 IU/kg to about 4,000 IU/kg of Vitamin D3 are added to swine feed. This addition improves the pig's bone strength.

Stark et al. (US 5,695,794) disclose adding a combination of 25-OH Vitamin D3 and Vitamin D3 to poultry feed to ameliorate the effects of tibial dyschondroplasia.

Borenstein et al US 5,043,170 discloses the combination of Vitamin D3 and either 1-alpha-hydroxycholecalciferol or 1alpha, 25-dihydroxycholecalciferol to improve egg strength and leg strength in laying hens and older hens.

Chung et al, WO 2007/059960 discloses that sows fed a diet containing both Vitamin D3 and 25-hydroxVitamin D3 had improved general health status, body frame, litter size and health, and other production parameters. Also a 25-OH D3 human food supplement is disclosed, but its dosage range, 5-15 micrograms per kg body weight, which equals to an extremely high daily dosage of 300-900 micrograms per human is very high.

### Tai et al 2008 Nutrition 24(3):2790285 discusses the associations between Vit D deficiency and circulating glucose and possibly insulin sensitivity. However, there is no teaching nor suggestion to administer Vit D in combination with 25-OHD3.

To our knowledge the prior art does not teach or suggest use of 25-hydroxy vitamin D3 as a medicament for humans to treat hyperglycemia. Forms and dosages of a composition provide desirable effects when a human is treated with effective amounts of vitamin D3 and 25-hydroxyvitamin D3 to normalize blood glucose. Other advantages and improvements are described below or would be apparent from the disclosure herein.

### BRIEF DESCRIPTION OF THE INVENTION

The invention provides 25-hydroxyvitamin D3 (25-OH D3) in combination with Vitamin D for use in maintaining healthy blood plasma glucose levels or to reduce blood glucose levels to healthy levels in a human.

It has been found that 25-hydroxyvitamin D3 (calcifediol) can be used as a medicament to reduce high blood glucose or to maintain blood glucose at a normal level in a human. The medicament further comprises vitamin D3 (cholecalciferol). The human may be any age, including children and juveniles, starting from birth to adulthood, and from 18 years to 80 years of age, or more than 80 years of age. Forms and dosages of a pharmaceutical composition, as well as processes for manufacturing medicaments, are also disclosed.

In a first aspect, a method of administering at least 25-hydroxyvitamin D3 to a human is provided. This allows blood glucose may be reduced to or maintained at a level which is closer to a normal level (e.g., less than 7 mmol glucose per liter plasma for a fasting adult). Vitamin D3 is administered together with or separately from 25-hydroxyvitamin D3. They may be administered once per day, once per week, or once per month.

In a another aspect, a pharmaceutical composition suitable for human use is provided which comprises vitamin D3, 25-hydroxyvitamin D3, and a pharmaceutically acceptable carrier in amounts to reduce blood glucose levels in a human.

Further aspects will be apparent from the following description and claims, and generalizations thereto.

### DETAILED DESCRIPTION OF THE INVENTION

As used throughout the specification and claims, the following definitions apply:
"Vitamin D" means either Vitamin D3 (cholecalciferol) and/or Vitamin D2 (ergocaciferol). Humans are unable to make Vitamin D2 (ergocalciferol), but are able to use it as a source of Vitamin D. Vitamin D2 can be synthesized by various plants and is often used in Vitamin D in supplements as an equivalent to Vitamin D.
"Vitamin D metabolite" means any metabolite of Vitamin D other than 25-hydroxy vitamin D3.
"25-OH D3" refers specifically to 25-hydroxyvitamin D3
"25-OH D" refers to the 25-hydroxylated metabolite of either Vitamin D2 or Vitamin D3 which is the major circulating form found in plasma.
"Prevent" is meant to include amelioration of the disease, lessening of the severity of the symptoms, early intervention, and lengthening the duration of onset of the disease, and not intended to be limited to a situation where the patient is no longer able to contract the disease nor experience any symptoms.

Vitamin D3 and 25-hydroxyvitamin D3 may be obtained from any source, and a composition thereof may be prepared using convenient technology. In general, crystals of vitamin D3, 25-hydroxyvitamin D3, or both (separately or together) are dissolved in an oil with heating and agitation. Preferably, the oil is transferred into a vessel and heated. Thereafter, vitamin D3, 25-hydroxyvitamin D3, or both are added to the vessel, while maintaining the temperature of the oil or increasing it over time. The composition is agitated to dissolve the crystals of vitamin D3, 25-hydroxyvitamin D3, or both. Prior to addition to the oil, the crystals may be reduced in size by milling and/or sieving, to enhance dissolving. The composition may be agitated by stirring, vessel rotation, mixing, homogenization, recirculation, or ultrasonication. Preferably, the oil may be heated in the vessel to a temperature from about 80°C to about 85°C, sized crystals are introduced into the vessel, and the contents are stirred to dissolve the crystals into the oil.

The "oil" may be any edible oil, lipid, or fat: e.g., babassu oil, coconut oil, cohune oil, murumyru tallow, palm kernel oil, or tucum oil. The oil may be natural, synthetic, semisynthetic, or any combination thereof. Natural oil may be derived from any source (e.g., animal, plant, fungal, marine); synthetic or semisynthetic oil may be produced by convenient technology. Preferably, the oil is a mixture of plant medium chain triglycerides, mainly caprylic and capric acids. The composition may optionally contain one or more other suitable ingredients such as, for example, antioxidants, preservatives, dissolution agents, surfactants, pH adjusting agents or buffers, humectants, and any combination thereof. The foregoing are examples of pharmaceutically acceptable carriers.

Suitable antioxidants include tocopherol, mixed tocopherols, tocopherols from natural or synthetic sources, butylated hydroxy toluene (BHT), butylated hydroxy anisole (BHA), natural antioxidants like rosemary extract, propyl galate, and any others used in the manufacture of pharmaceuticals for humans. Preferably, the antioxidant is tocopherol. Suitable preservatives include methyl paraben, propyl paraben, potassium sorbate, sodium benzoate, benzoic acid, and any combination thereof. Suitable dissolution agents include inorganic or organic solvents: e.g., alcohols, chlorinated hydrocarbons, and any combination thereof. Suitable surfactants may be anionic, cationic, or nonionic: e.g., ascorbyl palpitate, polysorbates, polyethylene glycols, and any combination thereof. Suitable pH adjusting agents or buffers include citric acid-sodium citrate, phosphoric acid-sodium phosphate, acetic acid-sodium acetate, and any combination thereof. Suitable humectants include glycerol, sorbitol, polyethylene glycol, propylene glycol, and any combination thereof.

Once formed, the oil composition may be incorporated in various other useful compositions, some of which are discussed below. For example, emulsions may be formed, which may be optionally encapsulated or spray dried. A variety of emulsions may be prepared by combining the nonaqueous compositions described above with an aqueous composition. The emulsion may be of any type. Suitable emulsions include oil-in-water emulsions, water-in-oil emulsions, anhydrous emulsions, solid emulsions, and microemulsions. The emulsions may be prepared by any convenient technology. The emulsion contains an aqueous composition and a nonaqueous (e.g., oil) composition, wherein the latter comprises vitamin D3, 25-hydroxyvitamin D3, or both (separately or together) dissolved in an oil in an amount of between about 3% and about 50% by weight based on the total weight of the oil composition. As used herein, "aqueous composition" and "aqueous phase" are used interchangeably. Generally, the emulsion may contain from about 20% to about 95% of an aqueous composition, and from about 5% to about 80% of a nonaqueous composition. Preferably, however, the emulsion contains from about 85% to about 95% (vol/vol) of an aqueous composition, and from about 5% to about 15% (vol/vol) of a nonaqueous composition. Conveniently, the nonaqueous composition may be dispersed as droplets in the aqueous composition. For example, the droplets may have a mean diameter of less than about 500nm in the aqueous composition. Conveniently, the droplets have a mean diameter of between about 100 nm and about 200 nm.

In a particularly advantageous embodiment, the emulsion contains an encapsulating agent, which facilitates encapsulating the oil composition upon further processing of the emulsion (e.g., by spray drying). The encapsulating agent may be any edible substance capable of encapsulating the oil composition. Preferably, the encapsulation agent is predominantly a colloidal material. Such materials include starches, proteins from animal sources (including gelatins), proteins from plant sources, casein, pectin, alginate, agar, maltodextrins, lignin sulfonates, cellulose derivatives, sugars, saccharides, sorbitols, gums, and any combination thereof.

Suitable starches include: plant starches (e.g., CAPSUL® or HI-CAP® from National Starch & Chemical Corp., New York, NY), other modified food starches, and any combination thereof. Preferably, the starch is CAPSUL® modified plant starch. Suitable proteins from animal sources include: gelatins (e.g., bovine gelatins, porcine gelatins (Type A or B) with different Bloom numbers, fish gelatins), skim milk protein, caseinate, and any combination thereof. Preferably, the animal protein is a gelatin. Suitable proteins from plant sources include: potato protein (e.g., ALBUREX® from Roquette Preres Societe Anonyme, Lestrem, France), pea protein, soy protein, and any combination thereof. Preferably, the plant protein is ALBUREX® potato protein. Suitable maltodextrins with a different dextrose equivalent include: maltodextrin 5, maltodextrin 10, maltodextrin 15, maltodextrin 20, maltodextrin 25, and any combination thereof. Preferably, the maltodextrin is maltodextrin 15. Suitable cellulose derivatives include: ethyl cellulose, methylethyl cellulose, hydroxypropyl cellulose, hydroxypropylinethyl cellulose, carboxymethylcellulose, and any combination thereof. Suitable saccharides include lactose, sucrose, or any combination thereof. Preferably, the saccharide is sucrose. Suitable gums include: acacia, locust bean, carragean, and any combination thereof. Preferably, the gum is gum acacia.

When the emulsion contains an encapsulating agent, the encapsulating agent may be dispersed in water by any convenient technology to form an aqueous phase. The aqueous phase may be a solution or a mixture depending on the properties of the components selected. The selected components may be dispersed by any convenient technology including: homogenizing, mixing, emulsifying, recirculating, static mixing, ultrasonication, stirring, heating, or any combination thereof. The viscosity of the resulting aqueous phase may then be adjusted, as desired, by the addition of water. The aqueous composition of the emulsion may optionally contain any other suitable material including but not limited to, those discussed above in reference to the nonaqueous composition. Preferably, the aqueous composition may include, an encapsulating agent, a film-forming agent, a plasticizer, a preservative, an antioxidant, or any combination thereof. Suitable preservatives include methyl paraben, propyl paraben, sorbic acid, potassium sorbate, sodium benzoate, and any combination thereof. Suitable antioxidants include sodium ascorbate, ascorbic acid, citric acid, and any combination thereof.

Preferably, the aqueous phase contains a modified food starch, such as octenyl succinyl starch (CAPSUL®), maltodextrin, and sodium ascorbate. Another preferred aqueous phase contains potato protein (ALBUREX®), maltodextrin 20, and sodium ascorbate. The selected components may be dissolved in water by any convenient technology, preferably stirring. The mixture is preferably homogenized until it is uniform and lump free. Preferably, the homogenization is carried out at a temperature between about 50°C and about 75°C. The final viscosity of the resulting aqueous phase may then be adjusted to the desired viscosity, preferably about 250 cp to about 450 cp, more preferably about 300 cp to about 400 cp, even more preferably about 385 cp.

The emulsion may be formed by emulsifying the nonaqueous composition and the aqueous phase by any means, including homogenization, rotor-stator shear, high pressure shear and cavitation, high speed "cowles" or shear agitation, and any combination thereof. The volume and viscosity of the emulsion may preferably be adjusted by the addition of water after emulsification. Preferably, the nonaqueous and aqueous compositions are emulsified by homogenization. Preferably, the emulsion should not contain any mineral, transition metal, or peroxide.

As noted above, the emulsion may be incorporated or employed in producing other useful compositions, especially encapsulated oils, e.g., spray-dried powders. Generally, the encapsulated oil comprises an oil composition and an encapsulation agent encapsulating the oil composition, wherein the oil composition contains vitamin D3, 25-hydroxyvitamin D3, or both dissolved in the oil in an amount between about 5% and about 50% by weight based on the total weight of the oil composition. The encapsulated oil may be produced by any convenient technology: e.g., drying an emulsion described above by any conventional technology, including spray drying, freeze drying, fluid bed drying, tray drying, adsorbtion, and any combination thereof. Preferably, the encapsulated oil is produced by spray drying an emulsion having an aqueous phase above containing an encapsulation agent; spray drying parameters are dictated by the physical characteristics desired in the final encapsulated oil. Such physical parameters include particle size, powder shape and flow, and water content. Preferably, the oil is in an amount less than about 30%, less than about 20%, less than about 10%, or less than about 5% by weight based on the total weight of the encapsulated oil. The encapsulated oil should have good flowability and the vitamin D3 and/or 25-hydroxyvitamin D3 should be distributed homogeneously throughout the composition. Conveniently, the encapsulated oil is a powder. Any other suitable additive may be added to the encapsulated oil. One such additive may be a flow agent such as silicon dioxide, to increase the flowability of the encapsulated oil.

### Dosages

*Daily.* A composition for use according to this invention where the two active ingredients are to be administered separately, or alone contains Vitamin D or 25-OH D3 in an amount from about 1 µg to about 50 µg, preferably about 5 µg and 25 µg. Alternatively, a single daily dosage having both Vitamin D and 25-OH D3 contains each active ingredient in an amount from about 1 µg to about 50 µg, preferably about 5 µg and 25 µg.

The dosage ratio of Vitamin D to 25-OH D3 may be from about 50:1 to about 1:50, more preferably from about 25:1 to about 1:25, and even more preferably from about 6:1 to about 1:6.

Multiple, separate dosages may be packaged in a single kit (or container). For example, the kit may be comprised of thirty separate daily dosages of both actives separately (i.e. 60 separate dosages), or combined (i.e. 30 dosages containing both active ingredients). Instructions for administering the dosages to a human may be included in the kit.

*Weekly.* A single weekly dosage contains Vitamin D or 25-OH D3 in an amount from about 7 µg to about 350 µg, and preferably from about 35 to 175 µg. Alternatively, a single weekly dosage may contain both Vitamin D and 25-OH D3 each in an amount from about 7 µg to about 350 µg, and preferably from about 35 to 175 µg. The dosage ratio of Vitamin D to 25-OH D3 may be from about 50:1 to about 1:50, more preferably from about 25:1 to about 1:25, and even more preferably from about 6:1 to about 1:6.

*Monthly.* A single monthly dosage contains Vitamin D or 25-OH D3 in an amount from 30 µg to about 1500 µg, preferably about 75 µg to about 500 µg. Alternatively, a single monthly dosage may contain both Vitamin D and 25-OH D3 each in an amount from 30 µg to about 1500 µg, preferably about 75 µg to about 500 µg. A kit may be comprised of one, two, three, four, five, six, seven, eight, nine, ten, eleven, or twelve weekly or monthly dosages.

Dosage ratios of Vitamin D to 25-OH D3 should range between 50:1 to about 1:50, more preferably from about 25:1 to about 1:25, and even more preferably from about 6:1 to about 1:6.

Blood glucose can be measured conveniently using an enzyme-linked assay to determine the amount of glucose in blood or fractions thereof (e.g., plasma and serum). Many different assays and devices are available to monitor blood glucose in diabetic humans. Glycosylated hemoglobin may be measured to monitor chronic hyperglycemia because hemoglobin is glycosylated when exposed to high levels of glucose over a prolonged time period. Hyperglycemia may result in symptoms such as pronounced hunger (polyphagia), excessive thirst (polydipsia), excessive urination (polyuria), fatigue, weight loss, and poor wound healing in diabetic humans. A normal level of blood (plasma) glucose is considered to be from about 4 mmol/L to about 7 mmol/L in fasting adults.

There is a scarcity of data on the relationship between orally-administered 25-hydroxyvitamin D3 and its resulting systemic concentration in humans, in comparison to orally-administered vitamin D3. The most comprehensive analysis to date of the kinetics of vitamin D3 and 25-hydroxyvitamin D3 was conducted by Barger-Lux et al. (Osteoperosis 8:222-230, 1998). Healthy men were administered up to 1250 µg/day of vitamin D3 over a period of eight weeks, and up to 50 µg/day of 25-hydroxyvitamin D3 over a period of four weeks. Curvilinear kinetics were demonstrated for the relationship of vitamin D3 and plasma 25-hydroxyvitamin D3, and it was suggested that this may be due to saturation of hydroxylase activity in the liver. This was supported in that dosing with 25-hydroxyvitamin D3 was not reported as producing curvilinear kinetics (Barger-Lux *et al.,* 1998). Although data on 25-hydroxyvitamin D3 does show curvilinear kinetics, it is only evident when the dose is extended past the level considered to result in maximum physiological benefit, which may indicate the activity of a homeostatic mechanism that is overwhelmed at very high doses. Within the physiological range, the relationship appears linear and comparable to Barger-Lux *et al.* These data indicate that a daily dose of between 10 µg and 60 µg of 25-hydroxyvitamin D is required for maximum health benefit.

A study of the pharmacokinetics in humans of orally-administered spray-dried 25-hydroxyvitamin D3, spray-dried vitamin D3, or both was initiated to investigate their physiological interactions. In particular, the shapes of their dose-response curves (which indicates the concentrations of vitamin D3 and 25-hydroxyvitamin D3 in the circulation over a set time course, not simply the average or maximum concentration achieved) and the steady-state kinetics were of interest. In respect of the former point, it is important to investigate the change in shape of the dose-response curves when exposure is to both vitamin D3 and 25-hydroxyvitamin D3. In respect of the latter point, it is also necessary to investigate their steady-state kinetics when dosing is less frequent than daily because this is the preferred regimen for groups that may have low compliance with taking daily supplements (such as the elderly).

The following non-limiting examples are presented to better illustrate the invention.

### Examples

### EXAMPLE 1

### CLINICAL TRIAL

### FORMULATION

### Materials and Methods

Spray-dried formulation of 25-hydroxyvitamin D3 was provided as a powder. In summary, 25-hydroxyvitamin D3 and DL-α-tocopherol were dissolved in an oil of medium chain triglycerides, then emulsified into an aqueous solution of modified starch, sucrose, and sodium ascorbate. The emulsion was atomized in a spray dryer in the presence of silicon dioxide. The resulting powder was collected when water content (LDO) was less than 4% and sieved through 400µm. It was packed and sealed in alu-bags, then stored in a dry area below 15°C and used within 12 months of its manufacture.

Three separate lots were manufactured. In detail, a matrix was produced by mixing for 120 min in a FRYMIX processing unit with an anchor stirrer at 70°C under vacuum and consisting of:
▪ 17.300 kg water (WBI)
▪ 13.460 kg modified food starch (CAPSUL HS)
▪ 3.270 kg sucrose
▪ 0.730 kg sodium ascorbate
An oil phase was prepared by mixing for 35 min in a double-walled vessel with propeller stirrer at 82°C and consisting of:
▪ 0.550 kg BERGABEST MCT oil 60/40
▪ 0.049 kg calcifediol (HY-D USP)
▪ 0.183 kg DL-α-tocopherol
The oil phase was transferred to the matrix in the FRYMIX processing unit and was pre-emulsified with its internal colloid mill (60 min, 70°C). The pre-emulsion was circulated through a high-pressure homogenizer (20 min). The emulsion with a viscosity of 60 mPa-s to 90 mPa·s s at 70°C was transferred over the high pressure pump to the spray nozzle. As fluidizing agent, silicon dioxide (SIPERNAT 320 DS) was fed into the tower. The spraying and drying parameters are listed below.

| **Parameter** | **Spraying** | **Drying** |
|---|---|---|
| Inlet air position | top of tower | top of tower |
| Inlet air feed | 1500 m³/h | 1400 m³/h |
| Inlet air temperature | 170 °C | heater switch off |
| IFB inlet air feed | 500m³/h | 500m³/h |
| IFB inlet air temperature | 65°C | 50°C |
| exhaust air position | bottom of the tower | bottom of the tower |
| fine powder recycling | to IFB | to IFB |
| emulsion feed rate | 50kg/h | emulsion feed stopped |
| SiO₂ feed position | top of tower | SiO₂ feed stopped |
| SiO₂ acid feed rate | 100g/h | SiO₂ feed stopped |

For each of the three lots of 25-hydroxyvitamin D3, an average of 8.4kg of spray-dried powder with about 0.25% content of 25-hydroxyvitamin D3 was obtained. The other components of the formulation are: 73.2% modified food starch, 17.6% sucrose, 4.0% sodium ascorbate, 3.0% medium chain triglycerides, 1.0% silicon dioxide, and 1.0% DL-α-tocopherol.

Spray-dried formulation of vitamin D3 was provided as a powder. In summary, vitamin D3 and DL-α-tocopherol were dissolved in an oil of medium chain triglycerides, then emulsified into an aqueous solution of modified starch, sucrose, and sodium ascorbate. The emulsion was atomized in a spray dryer in the presence of silicon dioxide. The resulting powder was collected when water content (LOD) was less than 4% and sieved to remove big lumps. It was stored in a dry area below 15°C and used within 12 months of its manufacture.

### Clinical trial

### Subjects

Healthy, postmenopausal women (50 to 70 years of age) were recruited using informed consent and screened using the following criteria: serum 25-hydroxy vitamin D3 between 20 nmol/L and 50 nmol/L, body mass index between 18 kg/m² and 27 kg/m², blood pressure less than 146/95 mm Hg, serum calcium less than 2.6 nmol/L, fasting glucose less than 100 mg/dl, no high-intensity exercise more than three times per week, no treatment for hypertension, no use of high-dose vitamin D or calcium supplement or drug affecting bone metabolism (e.g., biphosphonate, calcitonin, estrogen receptor modulator, hormone replacement therapy, parathyroid hormone), and not visiting a "sunny" location during the study.

Subjects were randomly assigned to one of seven treatment groups (i.e., daily, weekly, bolus as single dose, and bolus as combination dose). Each group included five subjects. They were followed for four months in Zurich, Switzerland during the winter.

### Design

The objective was studying and comparing the pharmacokinetic characteristics of vitamin D3 and 25-hydroxyvitamin D3 administered to humans. Equimolar quantities of both substances were investigated. The regimen was based on 20 µg/day (or its equivalent on a weekly basis) of 25-hydroxyvitamin D3. For comparative purposes, it was necessary to administer equimolar quantities of either vitamin D3 or 25-hydroxyvitamin D3. In respect to administration of vitamin D3, the dose was considered to be sufficient to overcome background variability and provide and efficacious dose to the participants.

**Daily: 120 administrations**

| | | |
|---|---|---|
| 1. | 25-Hydroxyvitamin D3 | 20 µg |
| 2. | Vitamin D3 | 20 µg (800 IU) |

| Weekly: 16 administrations | | |
|---|---|---|
| 3. | 25-Hydroxyvitamin D3 | 140 µg |
| 4. | Vitamin D3 | 140 µg (5600 IU) |

| Bolus: single administration | | |
|---|---|---|
| 5. | 25-Hydroxyvitamin D3 | 140 µg |
| 6. | Vitamin D3 | 140µg (5600 IU) |

| Bolus: combo administration | | |
|---|---|---|
| 7. | D3 and 25(OH)D3 | 140 µg (5600IU) + 140µg |

Hard gel capsules, which are packaged in bottles, contain either 20 µg or 140 µg of either spray-dried vitamin D3 or 25-hydroxyvitamin D3 per capsule. Each dosage was consumed orally at breakfast. The duration of the study was four months for the "Daily" and "Weekly" groups. Subjects enrolled in the "Bolus" group consumed orally a single dosage at the second study visit.

Plasma concentrations of 25-hydroxyvitamin D3 (e.g., peak and steady state) were determined by obtaining samples from the subjects at various times after the dosage was ingested. For screening purposes and to establish baseline values, a blood sample was obtained prior to enrollment into the study and the clinical laboratory measured vitamin D3, 25-hydroxyvitamin D3, calcium, creatinine, albumin, and fasting glucose in the serum. On Monday of Week 1 of the study, pharmacokinetics of serum vitamin D3, 25-hydroxyvitamin D3, and 1,25-dihydroxy vitamin D3; serum markers (i.e., vitamin D3, 25-hydroxyvitamin D3, calcium, creatinine, albumin, PTH, GOT, GPT, ALP, triglycerides, HDL, LDL, total cholesterol, bALP, and fasting glucose); and urine markers (i.e., calcium, creatinine, and DPD) were assessed over 24 hours. Daily samples for the remaining days of Week 1 and Monday of Week 2 were taken to assess serum vitamin D3 and 25-hydroxyvitamin D3, serum markers (i.e., calcium, creatinine, albumin), and urine markers (i.e., calcium, creatinine). The assessments continued on Monday of Weeks 3, 5, 7, 9, 11, 13 and 15. On Monday of Week 16, samples were taken to assess pharmacokinetics of serum vitamin D3, 25-hydroxyvitamin D3, and 1,25-dihydroxy vitamin D3; serum markers (i.e., vitamin D3, 25-hydroxyvitamin D3, calcium, creatinine, albumin, PTH, GOT, GPT, ALP, triglycerides, HDL, LDL, total cholesterol, bALP, and fasting glucose); and urine markers (i.e., calcium, creatinine, and DPD).

### RESULTS

Rigorous statistical analysis of the results obtained could not be performed for two reasons. First, upon unblinding the data, it was discovered that there was a difference between the groups' baseline glucose levels, and that this difference continued throughout the study. Secondly, upon examining individual's insulin levels, it was noted that a number of them were recorded as "0.0" in at least one visit. This indicates that there was either a problem with the analytics, the sample, the method or a combination.

However, despite the above problems, the following observations were made.

### Glucose levels:

Of the ten individual receiving daily or weekly Vitamin D3, 7 had lower glucose levels at week 15 as compared to week 2. For 25-OH D3, 6 of 10 had lower glucose levels. Thus, it appears that both Vitamin D3 and 25-OH can lower glucose levels.

### Insulin levels:

Of the ten individuals receiving daily or weekly Vitamin D3, only four had insulin levels which did not include a "0.0" result in any visit. Of these four, three had higher insulin levels at week 15 than at week 2; and only one had a lower value.

Of the ten individuals receiving daily or weekly 25-OH D3, five had insulin levels which did not include a "0.0" in any visit. Of these five, one had a higher insulin level at week 15 compared to week 2; but four had a lower level.

A lower insulin level is a desired result, as it indicates that insulin sensitivity is improved. Thus, it appears that 25-OH D3 has a better ability to improve insulin sensitivy than does Vitamin D3.

### Example 2

### Mouse study

The effects of 25-OH D3 or the combination of 25-OH D3 and Vitamin D3 on blood glucose were tested in two studies in mice.

In the first study, the effects of 25-OH D3 on blood glucose were determined in a model of muscle hypertrophy. Briefly, two groups of 10 animals were anesthetized and the left hindlimb of the animals was fixed. All animals received an analgesic. A small incision was made through the skin over the gastrocnemius muscle. The complete gastrocnemius muscle and his tendons were exposed. Both heads of the gastrocnemius muscle were carefully dissected from the underlying intact muscles and care was taken not to rupture nerves and vessels. The skin was closed with a silk suture and the animals were returned into the cages. After recovering from anesthesia the animals could move directly without problems in their cages. Animals were treated for three weeks by gavage with 25-OH D3 at a daily dosage of 50 µg/kg and the control group received vehicle. At the end of the study, blood was taken and plasma glucose concentration was analyzed by a Hitachi 912 Automatic Analyser.

In the second study, the effects of 25-OH D3 or the combination of 25-OH D3 and Vitamin D3 on blood glucose were tested in a model of muscle atrophy. Briefly, nine month old animals were randomized at the beginning of the study into five groups with 10 animals per group. The animals were placed in special cages for duration of three weeks and their tails were suspended, which leads to skeletal muscle atrophy of the unloaded hindlimbs. An additional group without hindlimb unloading was placed in identical cages in order to detect the effects of unloading. All mice were housed separately and had free access to feed and water ad libidum. All animals were treated daily by gavage throughout the 3 weeks of the experiment:
1. Control group without unloading received vehicle (gelatine)
2. Control group with unloading received vehicle (gelatine)
3. The Vitamin D3 group with unloading received Vitamin D3 (50 µg/kg/bw)
4. The 25-OH D3 group with unloading received 25-OH D3 (50 µg/kg/bw)
5. The 25-OH D3 plus Vitamin D3 group with unloading received Vitamin D3 + 25-OH D3 (50 + 50 µg/kg/bw)

Table 1 shows the plasma glucose values after completion of study 1.

**TABLE 1**

| | **Plasma glucose (in mmol/L)** |
|---|---|
| Control group | 15.53 |
| 25-OH D3 group | 14.65 |

Table 2 shows the plasma glucose values after completion of study 2.

**TABLE 2**

| | **Plasma glucose (in mmol/L)** |
|---|---|
| Control group without unloading | 12.02 |
| Control group with unloading | 12.89 |
| Vitamin D3 group with unloading | 12.69 |
| 25-OH D3 group with unloading | 12.75 |
| 25-OH D3 plus Vitamin D3 group with unloading | 12.21 |

The results from TABLE 1 indicate that treatment with 25-OH D3 resulted in a decrease in plasma glucose compared to the untreated control group. The results in TABLE 2 demonstrate that hindlimb unloading results in an increase in blood glucose levels (control group without unloading versus control group with unloading). Treatment with Vitamin D3 or 25-OH D3 resulted in a moderate decrease in blood glucose levels. However, treatment with the combination of Vitamin D3 and 25-OH D3 resulted in a synergistic decrease in plasma glucose levels which almost reached the level of the control animals without unloading, thus, ameliorating the effect of unloading. Therefore, our data show that the combination of Vitamin D3 and 25-OH D3 synergistically decreases elevated blood glucose levels and normalizes pathologically changed glucose levels.

## Claims

1. 25-hydroxyvitamin D3 (25-OH D3) in combination with Vitamin D for use in maintaining healthy blood plasma glucose levels or to reduce blood glucose levels to healthy levels in a human.

2. 25-OH D3 in combination with Vitamin D for use according to Claim 1 wherein the blood glucose levels are at a level between 4 mmol/L and 7 mmol/L, or are reduced to a level between 4 mmol/L and 7 mmol/L.

3. 25-OH D3 in combination with Vitamin D for use according to Claim 1 or 2 wherein the human is an adult.

4. 25-OH D3 in combination with Vitamin D for use according to any of Claims 1-3 wherein the Vitamin D3 is administered separately from the 25-OH D3.

5. 25-OH D3 in combination with Vitamin D for use according to any of Claims 1-4 wherein the ratio of Vitamin D3 to 25-OH D3 is from 1:50 to 50:1; preferably 1:25 to 25:1; and more preferably 6:1 to 1:6.

6. 25-OH D3 in combination with Vitamin D for use according to any of Claims 1-5 wherein the 25-OH D3 is in the form of a daily dose, a weekly dose, or a monthly dose.

## Patentansprüche

1. 25-Hydroxyvitamin D3 (25-OH D3) in Kombination mit Vitamin D zur Verwendung bei der Aufrechterhaltung gesunder Blutplasmaglucosespiegel oder zur Reduktion von Blutglucosespiegeln auf gesunde Niveaus bei einem Menschen.

2. 25-OH D3 in Kombination mit Vitamin D zur Verwendung nach Anspruch 1, wobei die Blutglucosespiegel auf einem Niveau zwischen 4 mmol/l und 7 mmol/l sind oder auf ein Niveau zwischen 4 mmol/l und 7 mmol/l reduziert werden.

3. 25-OH D3 in Kombination mit Vitamin D zur Verwendung nach Anspruch 1 oder 2, wobei es sich bei dem Menschen um einen Erwachsenen handelt.

4. 25-OH D3 in Kombination mit Vitamin D zur Verwendung nach einem der Ansprüche 1-3, wobei das Vitamin D3 separat vom 25-OH D3 verabreicht wird.

5. 25-OH D3 in Kombination mit Vitamin D zur Verwendung nach einem der Ansprüche 1-4, wobei das Verhältnis von Vitamin D3 zu 25-OH D3 1:50 bis 50:1, vorzugsweise 1:25 bis 25:1 und besonders bevorzugt 6:1 bis 1:6 beträgt.

6. 25-OH D3 in Kombination mit Vitamin D zur Verwendung nach einem der Ansprüche 1-5, wobei das 25-OH D3 in Form einer Tagesdosis, einer Wochendosis oder einer Monatsdosis vorliegt.

## Revendications

1. 25-hydroxy-vitamine D3 (25-OH D3) en combinaison avec de la vitamine D pour une utilisation à des fins de maintien de taux de glucose dans le plasma sanguin sains ou à des fins d'abaissement de taux de glucose sanguins jusqu'à des niveaux sains chez un être humain.

2. 25-OH D3 en combinaison avec de la vitamine D pour une utilisation selon la revendication 1, les taux de glucose sanguins étant à un niveau situé entre 4 mmol/L et 7 mmol/L, ou étant abaissés à un niveau situé entre 4 mmol/L et 7 mmol/L.

3. 25-OH D3 en combinaison avec de la vitamine D pour une utilisation selon la revendication 1 ou 2, l'être humain étant un adulte.

4. 25-OH D3 en combinaison avec de la vitamine D pour une utilisation selon l'une quelconque des revendications 1 à 3, la vitamine D3 étant administrée séparément vis-à-vis de la 25-OH D3.

5. 25-OH D3 en combinaison avec de la vitamine D pour une utilisation selon l'une quelconque des revendications 1 à 4, le rapport de la vitamine D3 à la 25-OH D3 étant de 1:50 à 50:1, de préférence de 1:25 à 25:1, et plus préférablement de 6:1 à 1:6.

6. 25-OH D3 en combinaison avec de la vitamine D pour une utilisation selon l'une quelconque des revendications 1 à 5, la 25-OH D3 se présentant sous la forme d'une dose journalière, d'une dose hebdomadaire ou d'une dose mensuelle.
